# EUROPEAN PATENT SPECIFICATION

(11) **EP 0 906 122 B1**
(45) Date of publication and mention of the grant of the patent: **10.09.2003**
(21) Application number: 96944251.6
(22) Date of filing: 06.12.1996
(51) Int. Cl.: A61K 47/48

(54) **ACTIVATED LINKERS AND METHODS FOR MAKING AND PURIFYING THE SAME**
AKTIVIERTE VERBINDUNGEN UND VERFAHREN ZUR HERSTELLUNG UND REININGUNG
SEGMENTS DE LIAISON ACTIVES ET LEURS PROCEDES DE FABRICATION ET DE PURIFICATION

(30) Priority: 06.03.1996 US 611918
(43) Date of publication of application: 07.04.1999
(73) Proprietor: Amgen Inc., Thousand Oaks, California 91320-1799 (US)
(72) Inventor: IBRAHIM, Prabha, N., Boulder, CO 80304 (US); BAILE, Robert, A., Rollinsville, CO 80403 (US); SEELY, James, Ervin, Louisville, CO 80027 (US)
(74) Representative: Brown, John David
(86) International application number: PCT/US96/19459
(87) International publication number: WO 97/032607

(56) References cited:
- EP-A- 0 622 394
- WO-A-92/16221
- WO-A-93/01498
- WO-A-95/13312
- WO-A-95/34326
- DATABASE BIOSIS BIOSCIENCES INFORMATION SERVICE, PHILADELPHIA, PA, US AN=95:239228, XP002040653 & SEELY J.C.R ET AL.: "MANUFACTURING OF RECOMBINANT TUMOR NECROSIS FACTOR BINDING PROTEIN "DUMBBELL" USING A 20K PEG BIS-SULFONE LINKER." ABSTRACTS OF PAPERS AMERICAN CHEMICAL SOCIETY, vol. 209, no. 1-2, 1995, page BIOT68

## Description

The present invention relates to methods of making PEG activated linkers, particularly activated linkers having reactive Michael acceptors, and to an efficient process for purifying the activated linkers and pegylated biologically active molecules.

### BACKGROUND OF THE INVENTION

Polyethylene glycols ("PEGs") are long chain, linear synthetic polymers composed of ethylene oxide units. The ethylene oxide units can vary such that PEG compounds can be obtained with molecular weights ranging from 200 - 100,000. PEG typically is colorless, odorless, soluble in water, stable to heat, does not hydrolyze or deteriorate, and is non-toxic. As such, PEGs have been extensively studied for use in pharmaceuticals, on artificial implants, and other applications where biocompatibility is of importance.

Various derivatives of PEG having an active moiety that permits PEG to be attached to various surfaces and molecules are known. For example, PEG derivatives have been used to couple PEG to surfaces to control wetting, static buildup, and attachment of other molecules to the surface. Specifically, PEG derivatives have been formed by reacting PEGs with a reagent having a reactive carbonyl, nitrile or sulfone group to convert the hydroxyl group into a reactive Michael acceptor, thereby forming an "activated linker" useful in modifying various proteins to provide improved biologically-active conjugates.

The attachment of PEG to proteins ("PEGylation") has been shown to: (1) increase the plasma half-life of the proteins, see e.g., U.S. Patent No. 5,349,052, Delgado et al., issued September 20, 1994; (2) alter the biochemical and physical properties of the protein, including increasing solubility, Chen et al., Biocem. Biophys. Acta, 660:293-298 (1988), and improve protection against proteolysis, Sada, et al., J. Fermentation Bioengineering 71:137-139 (1991); and (3) reduce antigenicity, Davis et al., "Biomedical Polymers. Polymeric Materials and Pharmaceuticals for Biomedical Use", (Eds. Goldberg & Nakajima) pp 441-452, Academic Press, N.Y (1980).

Problems have been encountered in attaching PEG to surfaces and molecules. The vast majority of PEGylating reagents react with free primary amino groups of the polypeptide, and most of these free amines are the ε-NH₂ group of lysine amino acid residues. Because typical proteins possess a large number of lysine residues, random attachment of multiple PEG molecules, i.e. non-specific PEGylation, often occurs. Non-specific PEGylation can lead to loss of protein activity, and, if the PEGylated protein is intended for therapeutic use, the multiple species that result from the use of non-specific PEGylation leads to difficulties in the preparation of a product with reproducible and characterizable properties. As such, non-specific PEGylation makes it difficult to evaluate therapeutics and to establish efficacy and dosing information. The site selective PEGylation of such proteins could lead to reproducibly-modified materials that gain the desirable attributes of PEGylation without the loss of activity.

Published PCT Publication No. WO 95/13312 describes, *inter alia*, water soluble sulfone-activated PEGs which are highly selective for coupling with thiol moieties instead of amino moieties on molecules and on surfaces. These PEG derivatives are stable against hydrolysis for extended periods in aqueous environments at pHs of about 11 or less, and can form linkages with molecules to form conjugates which are also hydrolytically stable. The linkage by which the PEGs and the biologically active molecule are coupled includes a sulfone moiety coupled to a thiol moiety and has the structure PEG-SO₂-CH₂-CH₂-S-W, where W represents the biologically active molecule, and wherein the sulfone moiety is vinyl sulfone or an active ethyl sulfone.

One example of a biologically active molecule which can be modified by such sulfone-activated linkers is TNF binding protein ("TNFbp") (also referred to as TNF inhibitor). TNFbp is of therapeutic interest in the treatment of TNF-mediated disease, including rheumatoid arthritis and septic shock. TNF binding proteins are disclosed in the art; see, e.g., EP 308 378, EP 393 438, EP 422 339, WO 90/13575, EP 398 327, EP 412 486, WO 91/03553, EP 418 014, JP 127,800/1991, EP 433 900, U.S. Patent No. 5,136,021, EP 512 528, EP 526 905, WO 93/07863, EP 568 928, WO 93/21946, No 93/19777, EP 417 563, GB 2,246,569, U.S. Provisional Patent Application No. 60/021443, filed July 9, 1996; and U.S. Provisional Patent Application No. , filed December 6, 1996.

PCT Publication No. WO 92/16221, incorporated herein by reference, describes TNFbp forms represented by the formula R₁-X-R₂ (referred to as a "dumbbell" compound), wherein R₁ and R₂ are biologically active TNFbp groups and X is a non-peptidic polymeric spacer with at least one Michael acceptor group, e.g. a sulfone-activated PEG. One particular compound, referred to as c105 TNFbp mutein dumbbell, is a homodumbbell compound wherein R₁ and R₂ are a c105 30kDa TNFbp mutein, X is 20K polyethylene glycol bis-vinylsulfone (20 kDa PEGbv), and wherein R₁ and R₂ are attached site-specifically at the cysteine 105 residue to the PEGbv.

A problem commonly encountered by those skilled in the art when producing mono, di, or polypegylated compounds is that the procedures used to synthesize the 20kDa PEGbv, or other activated linker, have several drawbacks, depending on the synthesis procedure used. For example, conventional methods for activating PEG with a functional group useful as linkers to attach to various molecules and surfaces are generally labor and time intensive. Such methods often require a multi-step reaction procedure to produce the desired product. Accordingly, a need exists for efficient methods for producing activated linkers that can be used to form desired conjugates.

Another particularly problematic drawback is that the resultant 20kDa PEGbv will often contain high molecular weight PEG impurities and PEG mono-vinylsulfone and PEG bis-vinylsulfone species. These impurities can then adversely affect the overall yield of the desired compound. The need also exists, therefore, for methods which would further purify the PEGbv, or other activated linkers, and thereby enhance the overall purity and yield of the desired compound.

A number of groups have reported on the use of reversed-phase high performance liquid chromatography (rpHPLC) to separate low- and medium-molecular weight (<5K) PEGs on the basis of size; see e.g., Murphy et al., J. Chromat., 211: 160-165 (1981); Lai et al., J. High Res. Chrom. & Chrom. Comm., 7: 494-496 (1984); Barka & Hoffman. J. Chromat., 389: 273-278 (1987). These techniques, while relatively effective, suffer from their use of expensive resins and organic solvents. Also, these methods are analytical rather than preparative procedures. There have been no reports of successful separation of larger molecular weight PEGs using HPLC. Medium-and high-molecular-weight (1K - 40K) PEGs have been analyzed by size exclusion chromatography on a variety of derivatized silica supports; Engelhardt & Mathes, J.Chromat., 185: 305 (1979).

Hydrophobic interaction chromatography (HIC) has achieved acceptance as an analytical and preparative purification method for biomolecules. The chemical principles underlying HIC are similar to those involved in salt precipitation, and related to those associated with reversed phase liquid chromatography (RPLC), in that all operate on the basis of hydrophobicity. HIC is characterized by adsorption of molecules to a weekly hydrophobic surface at high salt concentrations, followed by elution with a decreasing salt gradient. Thus, HIC combines the non-denaturing characteristics of salt precipitation and the precision of chromatography to yield excellent activity recoveries. Moreover, because HIC operates at lower binding energy, it does not require the use of expensive organic solvents in the mobile phase.

To date there have been no reports concerning the use of HIC resins to separate PEGs, especially high molecular weight PEGs. The skilled artisan would not expect HIC to be effective in separating PEGs based on size, as PEGs have the same hydrophobicity on a relative basis, regardless of size.

### SUMMARY OF THE INVENTION

The present invention provides novel methods of making and purifying activated polyethylene glycol linkers useful for a variety of purposes.

According to one aspect of the present invention, there is provided a method for making an activated polyethylene glycol linker comprising the steps of:
(a) obtaining a polyethylene glycol polymer (PEG) having a reactive hydroxyl group;
(b) converting said hydroxyl group to a reactive Michael acceptor to produce at least one form of an activated linker;
(c) applying said activated linker to a hydrophobic interaction chromatography (HIC) column that utilizes polymeric resin having a hydrophobic pendant group located on the surface of a hydrophilic vinyl copolymer backbone, wherein said hydrophobic pendant group is selected from octyl, butyl, phenyl or ether; and
(d) selectively isolating an activated linker form by utilizing elution conditions which allow for separation of various forms based on size and/or end-group functionality.

According to a second aspect of the present invention, there is provided a process for the preparation of a pegylated biologically active molecule, active said process comprising:
(a) steps (a) to (d) above.
(b) reacting the HIC-purified PEG, obtained according to steps (a) to (d) above, with a biologically active molecule to produce a pegylated biologically active molecule.

Preferably, said pegylated biologically active molecule has the formula R₁-X-R₂, wherein: X comprises an HIC-purified PEG having a first reactive group and a second reactive group, and wherein said first reactive group is a Michael acceptor; R₁ comprises a molecule having a reactive thiol moiety, said thiol moiety being covalently bonded with said Michael acceptor; and R₂ comprises a molecule or a nonbiologically-active group bonded to said PEG by reaction with said second reactive group. Further preferably, X is polyethylene glycol-bis-vinyl sulfone and wherein R₁ and R₂ is c105 30kDa TNF inhibitor.

Briefly, the methods of making the activated linkers of the present invention may be accomplished by obtaining a PEG having a reactive hydroxyl group and converting the hydroxyl group to a reactive Michael acceptor to form an activated linker, with the use of tetrahydrofuran (THF) as the solvent for the conversion. The process for purifying the activated linkers utilizes HIC to separate the linkers based on size and end-group functionality.

More particularly, the invention is directed to a method of making and purifying 20kDa polyethylene glycol bis-vinylsulfone ("20kDa PEGbv") which utilizes a Butyl or Phenyl HIC resin to separate high molecular weight PEG impurities and to separate PEG mono-vinylsulfone from the PEG bis-vinylsulfone. Importantly, purifications carried out in this manner do not require the addition of organic solvents. More importantly, the purified activated linkers can be used to modify various proteins and provide biologically-active conjugates with dramatically improved purity and overall yield as compared to conjugates formed with non-purified activated linkers.

Compounds having a reactive hydroxy group are polyethylene glycols polymers (PEGs). Of these, polyethylene glycol having a molecular weight in the range of 2kDa to 100kDa daltons are particularly useful, especially PEGs having a molecular weight of 3.4kDa, 5kDa, 10kDa or 20kDa. Such PEGs can be in the form of PEG diols.

Useful reagents having a reactive sulfone group that can be used in the methods include, without limitation, vinylsulfone and divinylsulfone. When used in conjunction with a PEG having one or two reactive hydroxy group(s), the methods produce PEG-vinylsulfone and PEG-*bis*-vinylsulfone, respectively. Such activated linkers can be used to make mono-pegylated compounds as well as dumbbell and multimeric compounds in which the linkers are capable of attaching more than one moiety. The addition of THF as the solvent at a ratio in the range of 1:10 to 1:50 (weight/volume) of PEG to THF has been found useful in the methods of the present invention. A ratio of 1:30 has been found particularly useful.

The biologically-active molecule contemplated for use can be a synthetic, a naturally occurring, or a modified naturally occurring molecule.

In one aspect of the invention, a PEG activated linker, having a molecular weight ranging from 2kDa to 100kDa, is applied to, and eluted from, an HIC resin using conventional chromatographic methods. In a preferred embodiment, the linker is eluted from the resin using a reverse linear salt gradient, such that the various linker forms are separated based on size and/or end-group functionality. Preferably, the linker is a sulfone-activated PEG linker and the HIC resin comprises a Butyl or Phenyl HIC resin. In a particularly preferred embodiment, the invention is directed to a process for purifying 20kDa polyethylene glycol vinylsulfone or bis-vinylsulfone ("20kDa PEGbv") utilizing a Toyopearl® Butyl-650M HIC resin.

In yet another aspect of the invention, the HIC-purified, PEG linker is reacted with a biologically-active molecule in order to provide a dumbbell compound having improved overall purity and yield as compared to previously described compounds containing the same molecule. Preferably, the HIC-purified polymer linker is a sulfone-activated PEG, and the biologically-active molecule is selected from the group consisting of TNF inhibitors, interleukin-1 receptor antagonists ("IL-1ra's"), exon six peptide of platelet-derived growth factor ("PDGF"), and interleukin-2 ("IL-2") inhibitors and receptors ("IL-2r"). In a particularly preferred embodiment, the HIC-purified polymer linker is 20kDa PEG vinylsulfone or PEGbv, and the biologically-active molecule is TNFbp.

Pharmaceutical compositions containing the compounds of the present invention are also included within the scope of the invention.

### BRIEF DESCRIPTION OF THE DRAWINGS

Figure 1 depicts the elution profile of various PEG diols from a ToyoPearl® Butyl 650M HIC column. PEG-containing fractions were detected with Nessler's reagent and the molecular weights confirmed by SEC HPLC as described in Example 3.
Figure 2 depicts the elution profile of 20kDa PEGbv and high molecular weight PEGbv from a ToyoPearl® Phenyl 650S HIC column (2 mg/mL bed volume loading). PEGbv concentration was determined by Nessler's reagent and the % molecular weight impurity was determined by SEC HPLC as described in Example 3.
Figure 3 depicts the elution profile of 20kDa PEGbv (2 mg/mL bed volume loading) from a ToyoPearl® Butyl 650M HIC column. PEG-containing fractions were detected with Nessler's reagent and turbidity measured at 600 nm.
Figure 4 depicts the elution profile of non-HIC-purified 20kDa PEGbv from a SEC-HPLC column. Comparative example.
Figure 5 depicts the elution profile of HIC-purified 20kDa PEGbv from a SEC-HPLC column. A ToyoPearl® Butyl 650M HIC column (8 mg/mL bed volume loading) was used for the HIC purification.
Figure 6 depicts a typical POROS™ HS cation-exchange elution profile for a TNFbp PEGylation mixture (the mixture utilized non-HIC-purified 20kDa PEGbv). The various TNFbp peaks are shown as indicated. Comparative example
Figure 7 depicts the POROS™ HS cation-exchange elution profile for a TNFbp PEGylation mixture (Comparative example). The PEGylation mixture was prepared as described in Example 5 and utilized ToyoPearl® Butyl 650M HIC-purified 20kDa PEGbv.
Figure 8 depicts the POROS™ HS cation-exchange elution profile for a TNFbp PEGylation mixture. The PEGylation mixture was prepared as described in Example 5 and utilized non-HIC-purified 20kDa PEGbv. Comparative example
Figure 9 depicts the elution profile of c105 TNFbp mutein dumbbell from a SP-Sepharose HP column. A₂₈₀ absorbance is plotted vs. time Comparative example. The c105 TNFbp mutein dumbbell was prepared using ToyoPearl® Butyl 650M HIC-purified 20kDa PEGbv.
Figure 10 depicts the elution profile of c105 TNFbp mutein dumbbell from a SP-Sepharose HP column. A₂₈₀ absorbance is plotted vs. time. The c105 TNFbp mutein dumbbell was prepared using non-HIC-purified 20kDa PEGbv. Comparative example
Figure 11 depicts the POROS™ HS cation-exchange elution profile for an "early eluting" fraction from a 20kDa PEGbv ToyoPearl® Butyl 650M HIC purification. The PEGylation mixture was prepared as described in Example 6.

### DETAILED DESCRIPTION

The methods of making and purifying the activated polyethylene glycols, are described in more detail in the discussion below and are illustrated by the examples provided below. The examples show various aspects of the invention and include results of the use of a "one-step" process for making a sulfone-activated PEG, 20kDa polyethylene glycol vinylsulfone or bis-vinylsulfone ("20kDa PEGbv"), and the use of Butyl and/or Phenyl HIC resins to separate high molecular weight PEG impurities. The results were surprising in that HIC also separated PEG mono-vinylsulfone from the 20kDa PEGbv, and the resultant purified 20kDa PEGbv, when used to PEGylate TNFbp, provided a biologically active conjugate having dramatically improved overall yield and purity as compared to conjugates prepared using non-HIC-purified 20kDa PEGbv, or 20kDa PEGbv synthesized using a multi-step procedure.

The methods of making the activated linkers of the present invention are accomplished by obtaining a PEG having a reactive hydroxyl group and converting the hydroxyl group to a reactive Michael acceptor to form an activated linker, with the use of tetrahydrofuran (THF) as the solvent for the conversion. Optionally, the PEG having a reactive hydroxyl group can first be dissolved in toluene and dried or evaporated prior to converting the hydroxyl group to a Michael acceptor. The conversion step is then accomplished by adding THF as the solvent to the dried compound. Although a variety of solvents, including dichloromethane (DCM), dimethyl formamide (DMF), and DMSO, can be used during the conversion step, THF is preferred. Since THF is a chelator of cations, its use as a solvent helps to chelate the base cation during the conversion reaction, leaving the negatively-charged oxygen for reaction with reagent containing a carbonyl, nitrile or sulfone group to form the Michael acceptor. Suitable bases for use in the methods include, for example, piperidine, pyridine, triethylamine, benzyl trimethyl ammonium hydroxide (Triton B), and potassium hydroxide. Strong bases that contain a cation are preferred. Examples of such strong bases include sodium or potassium *tert*-butoxide, sodium or potassium alkoxides, and metal amides as well as sodium or potassium hydrides.

Next, a reagent having a reactive carbonyl, nitrite or sulfone group is added, followed by the addition of a base. The activated linkers that are formed by these methods can then be precipitated with the use of a suitable solvent and isolated by any means known in the art, including filtration, to obtain purified activated linkers. Suitable solvents include, for example, ethers, particularly diethyl ether and dipropyl ether, alcohols, hexane, xylene and the like.

The methods of making the activated linkers of the present invention are referred to as "one-step" processes as compared to the known processes requiring multi-steps. The one-step process that can be depicted, for example, as follows:

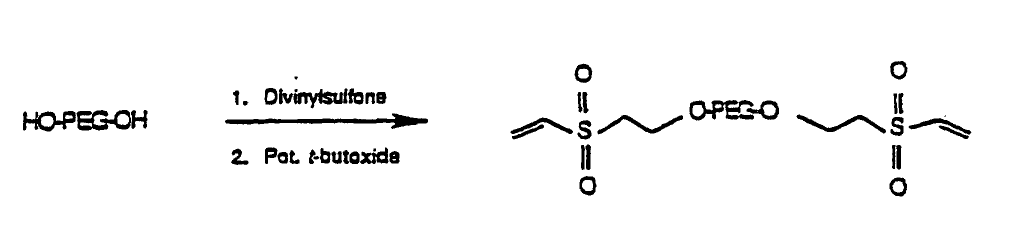

A "reactive hydroxyl group" means an -OH group in which the hydrogen can reduce the base, leaving the oxygen free to react with a reagent having a carbonyl, nitrile or sulfone group to form the Michael acceptor.

The PEG can be of any length or molecular weight but these characteristics can affect the biological properties. Useful polymer average molecular weights for decreasing clearance rates in pharmaceutical applications are in the range of 2,000 to 35,000 daltons. Particularly useful PEG molecular weights include 3.4kDa, 5kDa, 10kDa and 20kDa.

As used herein, the term "PEG" means any of several condensation polymers of ethylene glycol. PEG is also known as polyoxyethylene, polyethylene oxide, polyglycol, and polyether glycol. PEG can also be prepared as copolymers of ethylene oxide and many other monomers. The end groups of the PEG can be derivatized in a variety of ways to include vinylsulfone moieties, aldehyde moieties, methoxy groups, or other types of reactive or nonreactive end-groups. For many pharmaceutical or biotechnical applications, substantially linear, straight-chain vinyl sulfone activated PEG will be used which is substantially unsubstituted except for the vinyl sulfone.

The hydroxyl group of such compounds are reacted with a reagent having a reactive carbonyl, nitrile or sulfone group to convert the hydroxyl group into a Michael acceptor. Any reagent having a reactive carbonyl, nitrile or sulfone group known to those skilled in the art are contemplated for use in the present methods. "Reactive carbonyl, nitrile or sulfone" means a carbonyl, nitrile or sulfone group to which a two carbon group is bonded having a reactive site for thiol-specific coupling on the second carbon from the carbonyl, nitrile or sulfone group at pH 9 or less.

In preferred embodiments of the present invention, the polymeric derivatives of the present invention have active sulfone moieties. "Active sulfone" means a sulfone group to which a two carbon group is bonded having a reactive site for thiol-specific coupling on the second carbon from the sulfone group. Examples of active sulfones include, but are not limited to, vinyl sulfone and activated ethyl sulfone. The sulfone-activated polymer can be further substituted as long as the thiol-specific reactivity at the second carbon is maintained at pH 9 or less.

Upon the addition of such reagents, the hydroxyl group is converted to reactive Michael acceptor. "Michael acceptors" or "Michael-like acceptors" are used interchangeably and mean functional groups susceptible to Michael addition. "Michael addition" involves a nucleophilic attack on an electrophilic center which is adjacent to a pi system, having an electronegative atom. Examples of pi systems having an electronegative atom include sulfoxide, sulfonyl, carbonyl and heterocyclic aromatics. The nucleophile adds to the electrophilic center.
Michael acceptors can be represented by the formula: 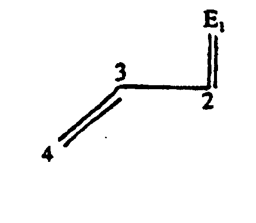 where E is an electronegative atom. Addition takes place at the 4 position to form the following: 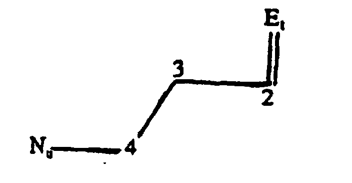 where Nᵤ represents the nucleophile now bonded to the atom at position 4. Michael acceptor functional groups include, but are not limited to, maleimide and vinyl sulfone. The activated linker used to form a dumbbell can, but need not, contain a vinyl sulfone species of Michael acceptor.

The activated linkers of the present invention can have more than one reactive group. The derivatives can be monofunctional, bifunctional, or multifunctional. The reactive groups may be the same (homofunctional) or different (heterofunctional) as long as there is at least one active sulfone moiety. Two particularly useful homobifunctional derivatives are PEG-*bis*-chlorosulfone and PEG-*bis*-vinylsulfone.

The resulting linkers produced by the methods of the present invention are stable, isolatable, and suitable for thiol-selective coupling reactions. For example, PEG chloroethyl sulfone is stable in water at a pH of 7 or less, but nevertheless can be used to advantage for thiol-selective coupling reactions at conditions of basic pH up to at least pH 9. At a pH of above 9, the thiol selectivity is diminished and the sulfone moiety becomes somewhat more reactive with amino groups. The linkage formed upon reaction with thiol is also hydrolytically stable.

As used herein, "hydrolytically stable" means that the linkage between the polymer and the sulfone moiety and between the sulfone-thiol after conjugation does not react with water at a pH of less than 11 for at least three days. Hydrolytic stability is desirable because, if the rate of hydrolysis is significant, the polymer can be deactivated before the reaction between polymer and the thiol of the biologically-active molecule takes place.

A general method for purifying the PEG activated linkers includes the following steps:
(1) synthesizing the activated linker;
(2) applying the activated linker to an HIC resin according to claim 1; and
(3) isolating the desired linker form by eluting the linker from the HIC resin using conditions, e.g., reverse linear salt gradient, which allow for separation of the various linker forms based on size and/or end-group functionality.

Alternatively, the HIC purification step could be incorporated into the "one-step" synthesis procedure just prior to the concentration/diafiltration step.

HIC contemplated for use in the present invention utilizes polymeric resins wherein a hydrophobic pendant group selected from octyl, butyl, phenyl or ether is located on the surface of a hydrophilic vinyl copolymer backbone resin, including, but not limited to, a methacrylic, agarose, or vinylic backbone. A silica based backbone could also be used. Preferred HIC columns will have either a butyl or phenyl ligand on a methacrylic backbone.

As stated above, the HIC-purified, sulfone-activated linkers can be attached to biologically active molecules that have a "reactive thiol moiety" to provide biologically active conjugates. A "reactive thiol moiety" means a sulfhydryl (-SH) group capable of reacting with the activated linkers as described herein.

Biologically-active molecules contemplated for use in the present invention include, but are not limited to, pharmaceuticals, vitamins, nutrients, nucleic acids, amino acids, polypeptides, enzyme cofactors, steroids, carbohydrates, organic species such as heparin, metal containing agents, receptor agonists, receptor antagonists, binding proteins, receptors or portions of receptors, extracellular matrix proteins, cell surface molecules, antigens, haptens, and chelating agents.

In general, TNFbp useful in the present invention has the sequence of human TNFbp, or truncated variants or muteins thereof. One TNFbp, referred to as 30kDa TNFbp, is the extracellular portion of the p55 TNF receptor. *In vivo*, the extracellular portion of the receptor is shed and circulates in the bloodstream as a 30kDa glycosylated protein which binds TNF. The purification and amino acid and nucleic acid sequences of this TNFbp are set forth in EP 393 438 and EP 422 339. These references also teach the recombinant production of glycosylated and deglycosylated forms of 30kDa TNFbp. Although the actual molecular weight of the deglycosylated form of this inhibitor is 18kDa, the term "30kDa TNF inhibitor" includes the glycosylated and deglycosylated forms.

The purification and amino acid and nucleic acid sequences of another TNF inhibitor, called 40kDa TNFbp, is also set forth in EP 393 438 and EP 422 339. This inhibitor, in its naturally-occurring form, is the glycosylated extracellular portion of the p75 TNF receptor. Although the molecular weight of the deglycosylated form is not 40kDa, both the glycosylated and deglycosylated forms of this TNFbp are referred to as "40kDa TNF inhibitor". These references also teach the recombinant production of glycosylated and deglycosylated forms of 40kDa TNFbp.

A reactive thiol group can be introduced into a biologically-active molecule when desirable. Thiol groups can also be introduced into an inactive molecule to form a biologically-active molecule as long as the thiol-sulfone link does not substantially destroy the desired activity.

Reactive thiol groups can be introduced by chemical means well known in the art. Chemical modification can be used with polypeptides or non-peptidic molecules and includes the introduction of thiol alone or as part of a larger group, for example a cysteine residue, into the molecule. An example of chemically introducing thiol is set forth in Jue, R. et al., *Biochemistry*, 17:5399-5406 (1978). One can also generate a free cysteine in a polypeptide by chemically reducing cystine with, for example, DTT.

To create cysteine muteins, a nonessential amino acid can be substituted with a cysteine or a cysteine residue can be added to the polypeptide. Polypeptides which are modified to contain an amino acid residue in a position where one was not present in the native protein before modification is called a "mutein." Potential sites for introduction of a non-native cysteine include glycosylation sites and the N or C terminus of the polypeptide. The mutation of lysine to cysteine is also appropriate because lysine residues are often found on the surface of a protein in its active conformation. In addition, one skilled in the art can use any information known about the binding or active site of the polypeptide in the selection of possible mutation sites.

One skilled in the art can also use well known recombinant DNA techniques to create cysteine muteins. One can alter the nucleic acid encoding the native polypeptide to encode the mutein by standard site directed mutagenesis. Examples of standard mutagenesis techniques are set forth in Kunkel, T.A., *Proc. Nat. Acad. Sci*., 82:488-492 (1985) and Kunkel, T.A. et al., *Methods Enzymol*., 154:67-382 (1987), both of which are incorporated herein by reference. Alternatively, one can chemically synthesize the nucleic acid encoding the mutein by techniques well known in the art. DNA synthesizing machines can be used and are available, for example, from Applied Biosystems (Foster City, CA). The nucleic acid encoding the desired mutein can be expressed in a variety of expression systems, including animal, insect, and bacterial systems.

The linkage between the biologically-active molecule having a thiol reactive group and the sulfone-activated polymer linker is a covalent bond, and the general method for preparing such conjugates includes the following steps:
(1) selecting the desired biologically-active molecule;
(2) synthesizing the desired sulfone-activated polymer linker;
(3) purifying the sulfone-activated polymer linker using a HIC chromatography according to claim 1;
(4) reacting the HIC-purified, sulfone-activated polymer linker with the biologically-active molecule;
(5) isolating the reaction product using chromatographic techniques well known in the art; and
(6) determining the biological activity of the conjugate formed using the relevant bioassay.

Muteins of the 30kDa TNFbp which have been prepared include: c105 30kDa TNFbp; c1 30kDa TNFbp; c14 30kDa TNFbp; c111 30kDa TNFbp; and c161 30kDa TNFbp. "c" refers to cysteine and "c105" means that a cysteine was inserted at position 105. These muteins have been prepared as set forth in published PCT Publication No. WO 92/16221, and the numbering is based upon the amino acid sequence set forth therein.

GB 2,246,569 (teaches a "3 Domain" 30kDa and/or 40kDA TNFbp, i.e., the fourth Domain is truncated); U.S. Provisional Patent Application No. 60/021443, filed July 9, 1996 (teaches a "2.6 Domain" 30kDa TNFbp, i.e., part of 3rd and 4th Domains are truncated); and U.S. Provisional Patent Application No. filed December 6, 1996, teaches a "2.6 Domain" 30kDa and/or 40kDa TNFbp, i.e., part of 3rd and 4th Domains are truncated.

Muteins of IL-1ra that have also been prepared include: c0s116 IL-1ra; c84s116 IL-1ra; c8s116 IL-1ra; c9s116 IL-1ra; and c141s116 IL-1ra. The preparation and purification of IL-1ra muteins are also set forth in published PCT Patent Publication No. WO 92/16221. The residue numbering is based upon the sequence set forth in that published application with "0" denoting addition of an amino acid at the N-terminus; "c" referring to cysteine and "s" referring to serine. For example, "c0s116" means a cysteine was inserted at the N terminus and a serine was inserted at position 116. Native IL-1ra has free cysteine residues at positions 66, 69, 116 and 122.

Pharmaceutical compositions containing many of the conjugates or compounds (collectively, the "conjugates") prepared according to the methods of the present invention can be prepared. These conjugates can be in a pharmaceutically-acceptable carrier to form the pharmaceutical compositions. The term "pharmaceutically acceptable carrier" as used herein means a non-toxic, generally inert vehicle for the active ingredient, which does not adversely affect the ingredient or the patient to whom the composition is administered. Suitable vehicles or carriers can be found in standard pharmaceutical texts, for example, in "Remington's Pharmaceutical Sciences", 16^{th} Ed., Mack Publishing Co., Easton, PA (1980). Such carriers include, for example, aqueous solutions such as bicarbonate buffers, phosphate buffers, Ringer's solution and physiological saline. In addition, the carrier can contain other pharmaceutically-acceptable excipients for modifying or maintaining the pH, osmolarity, viscosity, clarity, color, sterility, stability, rate of dissolution, or odor of the formulation.

The pharmaceutical compositions can be prepared by methods known in the art including, by way of an example, the simple mixing of reagents. Those skilled in the art will know that the choice of the pharmaceutical carrier and the appropriate preparation of the composition depend on the intended use and mode of administration.

In one example, it is envisioned that the carrier and the conjugate constitute a physiologically-compatible, slow-release formulation. The primary solvent in such a carrier can be either aqueous or non-aqueous in nature. In addition, the carrier can contain still other pharmacologically-acceptable excipients for modifying or maintaining the stability, rate of dissolution, release, or absorption of the conjugate. Such excipients are those substances usually and customarily employed to formulate dosages for parenteral administration in either unit dose or multi-dose form.

Once the pharmaceutical composition has been formulated, it can be stored in sterile vials as a solution, suspension, gel, emulsion, solid, or dehydrated or lyophilized powder. Such formulations can be stored either in a ready to use form or in a form requiring reconstitution immediately prior to administration. The preferred storage of such formulations is at temperatures at least as low as 4°C and preferably at -70°C. It is also preferred that such formulations containing the conjugates are stored and administered at or near physiological pH. It is presently believed that administration in a formulation at a high pH (i.e. greater than 8) or at a low pH (i.e. less than 5) is undesirable.

The manner of administering the formulations containing the conjugates for systemic delivery can be via subcutaneous, intramuscular, intravenous, oral, intranasal, or vaginal or rectal suppository. Preferably the manner of administration of the formulations containing the conjugates for local delivery is via intraarticular, intratracheal, or instillation or inhalations to the respiratory tract. In addition it may be desirable to administer the conjugates to specified portions of the alimentary canal either by oral administration of the conjugates in an appropriate formulation or device.

For oral administration, the conjugate is encapsulated. The encapsulated conjugate can be formulated with or without pharmaceutically-acceptable carriers customarily used in the compounding of solid dosage forms. Preferably, the capsule is designed so that the active portion of the formulation is released at that point in the gastro-intestinal tract when bioavailability is maximized and pre-systemic degradation is minimized. Additional excipients can be included to facilitate absorption of the conjugate. Diluents, flavorings, low melting point waxes, vegetable oils, lubricants, suspending agents, tablet disintegrating agents, and binders can also be employed.

Regardless of the manner of administration, the specific dose is calculated according to the approximate body weight of the patient. Other factors in determining the appropriate dosage can include the disease or condition to be treated or prevented, route of administration and the age, sex and medical condition of the patient. In certain embodiments, the dosage and administration is designed to create a preselected concentration range of the conjugate in the patient's blood stream. For example, it is believed that the maintenance of circulating concentrations of TNF inhibitor and IL-1 inhibitor of less than 0.01 ng per mL of plasma may not be an effective composition, while the prolonged maintenance of circulating levels in excess of 10 µg per mL may have undesirable side effects. Further refinement of the calculations necessary to determine the appropriate dosage for treatment involving each of the above mentioned formulations is routinely made by those of ordinary skill in the art and is within the ambit of tasks routinely performed by them without undue experimentation, especially in light of the dosage information and assays disclosed herein. These dosages may be ascertained through use of the established assays for determining dosages utilized in conjunction with appropriate dose-response data.

It should be noted that the conjugate formulations described herein may be used for the preparation of veterinary as well as human medicaments and that the term "patient" should not be construed in a limiting manner. In the case of veterinary applications, the dosage ranges should be the same as specified above.

The conjugates of the present invention can be used for a variety of purposes including, but not limited to, in-vitro diagnostic assays and the preparation of pharmaceutical compositions. Conjugates containing TNFbp, IL-1ra, alone or in combination can be used to treat diseases such as adult respiratory distress syndrome, pulmonary fibrosis, arthritis, psoriasis, myelogenous and other leukemias, ischemia/reperfusion, asthma, cachexia/anorexia, diabetes, septic shock, inflammatory bowel disease, multiple sclerosis, stroke, Crohn's Disease, graft rejection and hemorrhagic trauma. The conjugates can also be used for a number of in vitro purposes, including affinity chromatography (partitioning) and diagnostic uses, which are readily known to those skilled in the art. For example, such conjugates can be attached to a solid surface and used to purify or detect ligands that specifically bind to receptors coupled to the activated linkers.

The following examples will illustrate in more detail the various aspects of the present invention.

### EXAMPLE 1

This example describes the preparation of the activated linker, 20kDa PEGbv, using the "One-step" synthesis procedure.

### "One-step" Synthesis of 20kDa PEGbv

Polyethylene glycol (PEG-DIOL™) was purchased from Shearwater Polymers Inc. (Birmingham, AL). Divinylsulfone, 1M potassium *t*-butoxide (in tetrahydrofuran (THF)), toluene (anhydrous, sure seal), di-*i*-propyl ether (99+%, inhibited with 25 ppm butylated hydroxy toluene (BHT)), and tetrahydrofuran (anhydrous containing 0.025% BHT as inhibitor, sure seal) were obtained from Aldrich Chemical Company (Milwaukee, Wisconsin) Dichloromethane (Omni Solvent) and diethyl ether (EM) were bought from VWR Scientific Company (Denver, Colorado). Anhydrous sodium sulfate powder was purchased from J. T. Baker (Phillipsburg, New Jersey).

A 22 L four-necked (one 45/50 and three 24/40 joints) round bottom flask was fitted with a thermometer, condenser, a rubber septa, and a variable speed mechanical stirrer and immersed in a water bath containing an immersion water heater. The flask was kept under positive argon pressure. The condenser was connected to a two-neck round-bottom flask through which vacuum line was attached. Polyethylene glycol (20kDa, 300g, 15 mmole) was weighed and transferred to the 22 L flask through a funnel and three liters of anhydrous toluene was cannulated while the mixture was stirring. The water bath temperature was set at 45°C and the stirring continued until the solution became clear (∼1 h). The solvent was evaporated under vacuum (7999,3 Pa) (∼60 mm of Hg) to dryness. The solid residue was dissolved in nine liters of anhydrous tetrahydrofuran (added *via* cannula). The water bath temperature was set at 25°C. Stirring was continued until the temperature of the solution became 25°C. Divinylsulfone (6 ml, 59.97 mmole) was added from a syringe over 15 min. The mixture was stirred (75 rpm) for 10 min. Potassium *t*-butoxide (1M in THF, 3 ml, 3 mmole) was added from a dropping funnel over a period of 25 min.

After the addition of the base, the reaction mixture was stirred at 25°C for six hours. The water bath was then heated to 40°C and the THF was evaporated (4,67.10⁴ Pa). (350 mm of Hg). One liter of 1:1 mixture of dichloromethane and tetrahydrofuran was added to the solid residue and stirred until a clear solution was formed. The solution was cooled to 25°C and chilled (-20°C). Di-*i*-propyl ether (1 X 5 liter) was added to the solution from a dropping funnel while the stirring was continued. The condenser connection was removed from the flask. A pressure filter funnel was connected to the flask through a flex needle and the precipitate transferred via the flex needle and filtered through the filter funnel (1 L, 70-100µ) under vacuum. The precipitate was split into two portions, transferred into 2 L Erlenmeyers and left stirring (magnetic stirrer) with one liter (each) of *i*-propyl ether for three hours. The precipitate was filtered (2 L, 70-100µ), washed with 800 ml of diethyl ether and dried under vacuum (1199,9 Pa) (5 mm of Hg) in a desiccator for 72 h. The yield was determined to be 278.6 g (91.6% recovery based on the weight of polyethylene glycol).

The product was transferred to an erlenmeyer flask (2 L) and a liter of dichloromethane (Omni Solvent) added. After all the product had dissolved the solution (in syrup form) was transferred to a 5 L drop bottom flask fitted with a mechanical stirrer. The Erlenmeyer flask was rinsed with another 500 ml of dichloromethane and the solution added to the drop bottom flask. The solution was then stirred for 5 min (65 rpm). Saturated sodium chloride (1 L) was added and stirred for 5 min (75 rpm). The mixture was allowed to settle till the two layers became distinct. The organic layer was withdrawn and kept in a separate container. This process was repeated three times and all the organic layers were combined.

The solution was then split into two portions (3 L each) and transferred into two Erlenmeyer flasks (4 L) and stirred for 16 hours with 500g of anhydrous sodium sulfate powder (J. T. Baker, Phillipsburg, New Jersey), at room temperature, using a magnetic stirrer (during this process each flask was completely covered with aluminum foil). The solution was filtered through a filter funnel (2 L, 70-100µ) and the filtrate transferred to a 22 L four-necked flask, the flask having been placed in a water bath (40°C). The solvent was evaporated under vacuum (285-360 mbar) and the residue (very viscous liquid) re-dissolved in a 1:1 mixture of dichloromethane and THF (1 L). The solution was then precipitated, filtered, and dried as previously described. The yield was determined to be 221.0 g (72.67% recovery based on the weight of polyethylene glycol).

### EXAMPLE 2

This example describes a series of loading and elution experiments designed to test the capability of Butyl, Phenyl and Ether HIC resins to bind 20kDa PEGbv.

### Assays Utilized

### 1. PEG Assay

PEG-containing fractions were detected with Nessler's reagent (Sigma/Aldrich). Typically, 0.01 mL of Nessler's reagent is added to a 0.25 mL aliquot of a column fraction in a glass test-tube. PEG-containing fractions formed milky precipitate upon addition of the Nessler's reagent.

### 2. Quantitative PEG Analysis

Nessler's reagent (1.5 mL) was added to a glass test-tube, followed by 18.75 microliters of the sample to be tested. The test-tubes were vortexed, allowed to stand for 10 - 15 minutes, and turbidity read at 600 nm vs. a blank containing Nessler's reagent alone (Beckman DU 70 Spectrophotometer). PEG was then quantitated from a standard curve made up of 1.5, 1.0, 0.75, 0.50 and 0.25 mg/mL PEG standard.

### Experimental Runs

5 mL prepacked ToyoPearl® Butyl, Phenyl and Ether 650C HIC columns (TosoHaas, Montgomeryville, PA) were used. Columns were loaded with 10 mg (2 mg/mL bed volume) 20kDa PEGbv in various concentrations of NaCl or sodium sulfate, and then eluted with water by gravity. All experiments were conducted at room temperature, and 0.5 - 1.0 mL fractions were collected by hand into 13 X 100 mM glass test-tubes. PEG was determined by the Nessler's assay. Table 1 sets forth the salt concentrations necessary for complete binding (i.e., no PEGbv detectable in the flowthrough) of 20K PEGbv to the various HIC resins.

**Table 1**

| Resin | NaCl Conc. | Sodium Sulfate Conc. |
|---|---|---|
| Phenyl 650C | 2.25 M | 0.35 M |
| Butyl 650C | 1.75 M | 0.25 M |
| Ether 650C | >> 2 M | >>0.5 M |

As indicated in Table 1, the Butyl 650C HIC resin has a slightly greater affinity for PEG than does the Phenyl 650C HIC resin, while the Ether 650C HIC resin did not bind any PEG when loaded at either 2 M NaCl or 0.5 M sodium sulfate.

### EXAMPLE 3

This example describes an experiment designed to test the capability of a Butyl HIC resin to separate various PEG diols based on size. PEG-containing fractions were determined using the PEG assay described above and the molecular weight of the PEGS were confirmed by SEC HPLC.

### Assays Utilized

### 1. Size-exclusion Chromatography (SEC) HPLC Assay

Size-exclusion chromatography of the HIC-purified PEG fractions was performed using a Toso-Haas G3000_{swxl} column (Synchrom, Inc., Linden, NJ). A 100 - 200 µl sample at 0.5 - 1.0 mg/mL is injected. The column was run at room temperature and the equilibrated in 10 mM sodium phosphate buffer, 150 mM NaCl, 0.004% sodium dodecyl sulfate (SDS), pH 6.5. The PEG was eluted from the column using a buffer containing 10 mM sodium phosphate, 150 mM NaCl, and 0.004% SDS, pH 6.5. In some experiments, a Refractive Index detecto (Bio-Rad, Hercules, CA) was used to monitor the PEG elution. In other experiments, the PEG was first derivatized with o-thiobenzoic acid so that the eluate could be monitored by UV absorbance at 254 nm.

### Experimental Runs

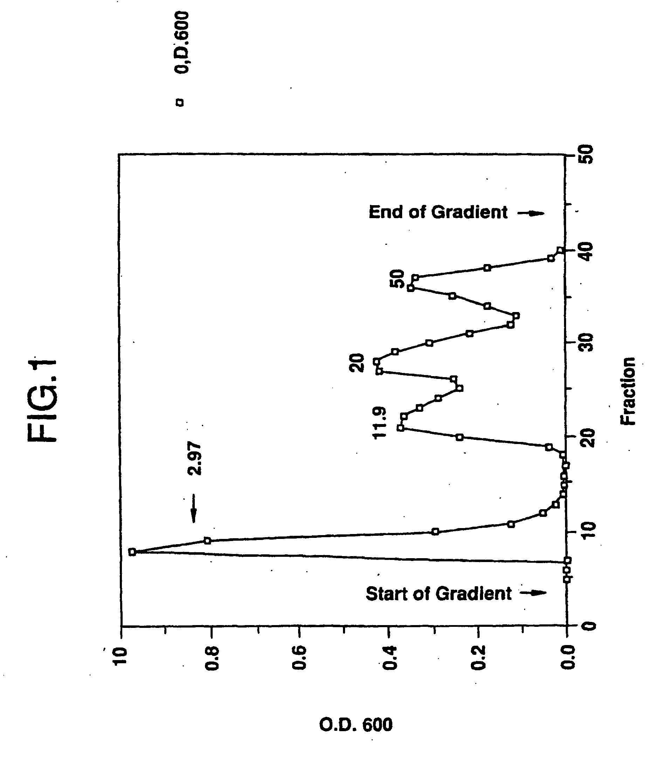

A ToyoPearl® Butyl 650M HIC column (1.5 X 11 cm) was loaded with a total of 3 mg PEG diol per mL column bed volume. The column was equilibrated in 4 M NaCl. The load volume was 9.7 mL and contained 2.97kDa, 11.9kDa, 20kDa and 50kDa PEG diol, each at 1.5 mg/mL (the 2.97kDa and 11.9kDa PEG diols were purchased from Polymer Laboratories and the 20kDa and 50kDa PEG diols were purchased from Shearwater Polymers). After loading, the column was washed with 2 column volumes of 4 M NaCl, and eluted with a linear 15 column volume gradient from 3 M to 1 M NaCl. The flow rate for load, wash and elution steps was 2.5 mL/min. Three minute (7.5 mL) fractions were collected and fractions were analyzed for PEG. The elution profile is shown in Figure 1. The molecular weights of the four peaks were confirmed by size-exclusion HPLC chromatography and are shown as indicated.

To complete these initial experiments, the PEGbv that eluted in the 2 M NaCl flow-through and water eluate of the Phenyl 650C column described in Example 2 was analyzed in the SEC HPLC assay. The SEC HPLC data revealed that the material in the 2 M NaCl flow-through was enriched for 20kDa PEGbv, whereas the material in the water eluate contained predominantly high molecular weight PEGbv. This data confirms that HIC; in particular, Phenyl and/or Butyl HIC, can be used to separate PEG diols and 20kDa PEGbv on the basis of size.

### EXAMPLE 4

This example describes a series of HIC experiments wherein various 20kDa PEGbv loading conditions were tested and wherein various linear NaCl gradients were used to elute the PEGbv from the Butyl or Phenyl HIC resins. All experiments were run at room temperature and generally carried out as described in Example 2.

### Run 1.

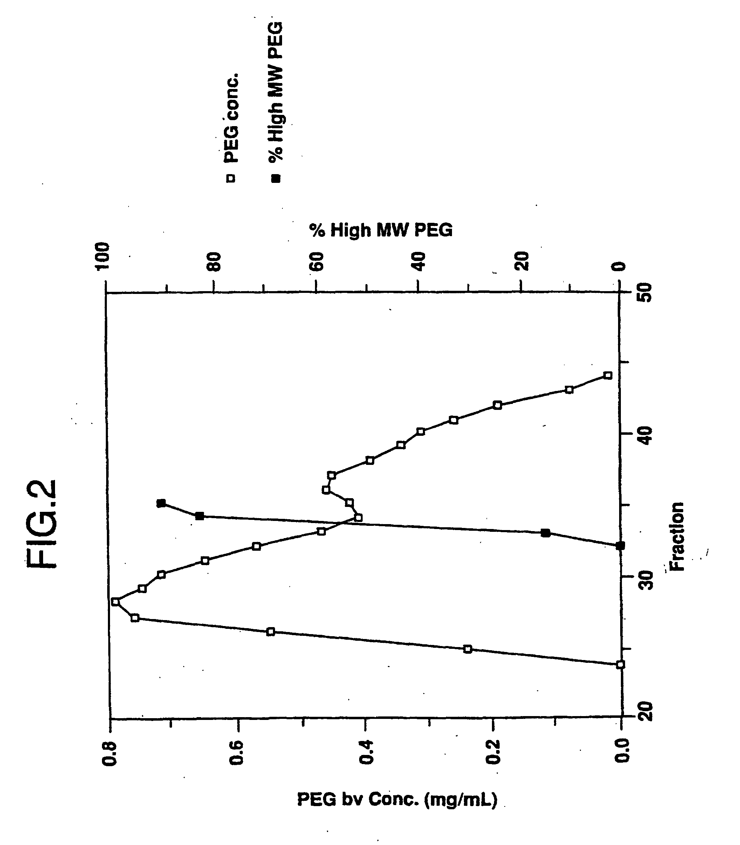

Run 1 used a ToyoPearl® Phenyl 650S HIC column (1.5 X 6.5 cm). The 6505 resin differs from the 650C resin in that the particle size is 20 - 50 µm (20 - 50 microns) rather than 50 - 150 µm (50 - 150 microns). The column was equilibrated in 3 M NaCl, and 2 mg/mL bed volume PEGbv was loaded onto the column. The PEGbv was eluted using a 10 column volume linear NaCl gradient (3 M NaCl to 0 M NaCl) and a flow-rate of 1.5 mL/minute. Two minute (3.0 mL) fractions were collected and the elution profile is depicted in Figure 2. The % yield of 20kDa PEGbv was then determined using the assay described in Example 3 (see Table 2).

### Run 2.

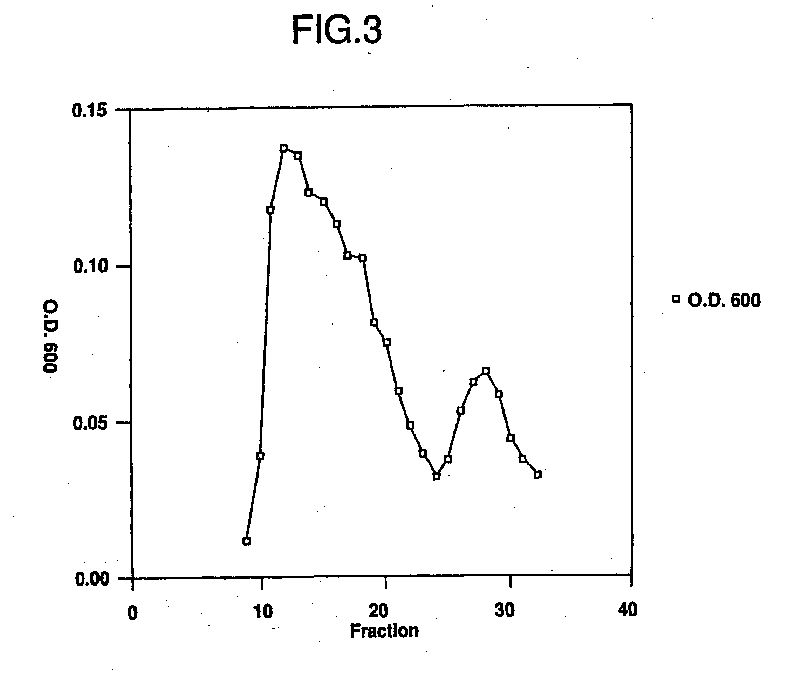

Run 2 used a ToyoPearl® Butyl 650M HIC column (1.5 X 11.4 cm). The 650M resin differs from the 650C resin in that the particle size is 40 - 90 µm (40 - 90 microns) rather than 50 - 150 µm (50 - 150 microns). The column was equilibrated in 2 M NaCl and 2 mg/mL bed volume PEGbv was loaded onto the column. The PEGbv was eluted using a 10 column volume linear NaCl gradient (2 M NaCl to 0 M NaCl) and a flow-rate of 1.5 mL/minute. Three minute (4.5 mL) fractions were collected and the elution profile is depicted in Figure 3. The % yield of 20kDa PEGbv was then determined using the assay described in Example 3 (see Table 2).

### Run 3.

Run 3 used a ToyoPearl® Butyl 650M HIC column (3.2 X 18.0 cm). The column was equilibrated in 2.5 M NaCl and 1.8 mg/mL bed volume PEGbv was loaded onto the column. The PEGbv was eluted using a 10 column volume linear NaCl gradient (2 M NaCl to 0.5 M NaCl) and a flow-rate of 9.6 mL/minute. 2.5 minute (24 mL) fractions were collected. The % yield of 20kDa PEGbv was then determined using the assay described in Example 3 (see Table 2).

### Run 4.

Run 4 used a ToyoPearl® Butyl 650M HIC column (1.5 X 11.5 cm). The column was equilibrated in 5 M NaCl and 8 mg/mL bed volume PEGbv was loaded onto the column. The increased bed volume loading was performed in order to more closely approximate running conditions at large scale. Because of the increased loading, a higher NaCl concentration (5 M vs. 2 M) was required for complete binding of PEGbv onto the column. The PEGbv was eluted using a 10 column volume linear NaCl gradient (3.5 M NaCl to 1 M NaCl) and a flow-rate of 2.0 mL/minute. 1.5 minute (3 mL) fractions were collected. The % yield of 20kDa PEGbv was then determined using the assay described in Example 3 (see Table 2).

### Run 5.

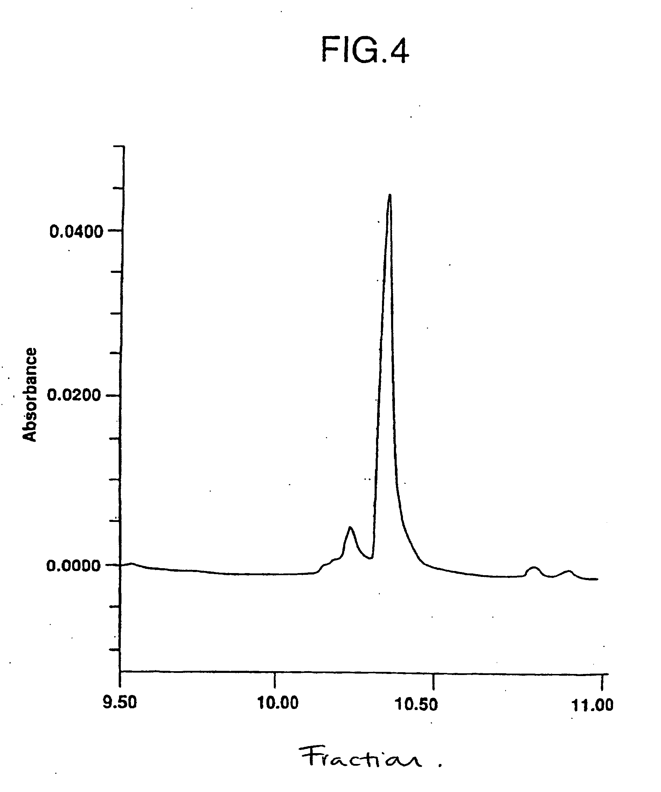

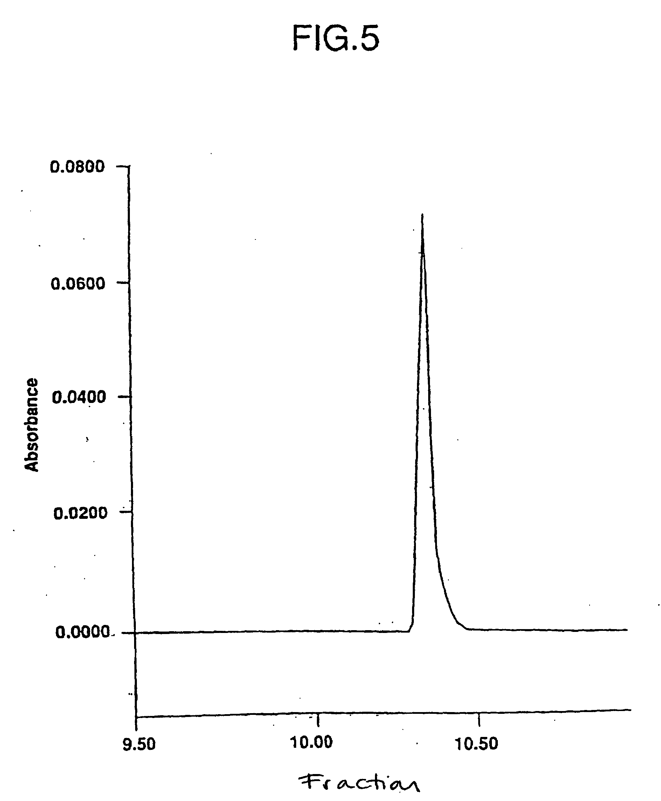

Run 5 used a ToyoPearl® Butyl 650M HIC column (1.5 X 11.5 cm). The column was equilibrated in 5 M NaCl and 8 mg/mL bed volume PEGbv was loaded onto the column. The PEGbv was eluted using a 15 column volume linear NaCl gradient (2 M NaCl to 0.5 M NaCl) and a flow-rate of 2.1 mL/minute. Fractions were collected and the elution profile is depicted in Figure 3. The resolution profile was not as good for this run as that obtained using 2 mg/mL loading; however, SEC HPLC purity obtained from the run was determined and is represented in Figures 4 and 5. The Figures 4 and 5 data demonstrates that, despite the poorer resolution, adequate removal of the high molecular weight PEGbv was achieved.

### Run 6.

Run 6 used a ToyoPearl® Butyl 650M HIC column (7.0 X 14 cm). The column was equilibrated in 4.5 M NaCl and 8 mg/mL bed volume PEGbv was loaded onto the column. The PEGbv was eluted using a 17 column volume linear NaCl gradient (3.5 M NaCl to 1.0 M NaCl) and a flow-rate of 55 mL/minute.

**Table 2**

| | Run #Resin | Load (mg PEGbv/ mL bed volume) | % yield 20kDa PEGbv |
|---|---|---|---|
| 1 | Phenyl 650S | 2 mg/mL | 75% |
| 2 | Butyl 650M | 2 mg/mL | 73% |
| 3 | Butyl 650M | 1.8 mg/mL | 64% |
| 4 | Butyl 650M | 8 mg/mL | 72% |
| 5 | Butyl 650M | 8 mg/mL | Not Determined |
| 6 | Butyl 650M | 8 mg/mL | Not Determined |

As indicated by the data above, the use of linear NaCl gradients in the Phenyl and/or Butyl HIC separations is effective in resolving high molecular weight PEGbv from the 20kDa PEGbv. Furthermore, a yield of approximately 65%-75% was obtained using a 2 mg/mL bed volume loading or an 8 mg/mL bed volume loading under appropriate conditions. Finally, the data indicate that, although the resolution profile is not as good for the 8 mg/mL loading runs as compared to the 2 mg/mL loading runs, the 8 mg/mL loading does achieve adequate removal of the high molecular weight PEGbv.

### EXAMPLE 5

This example describes the preparation of a biologically-active conjugate (homodumbbell) comprising TNFbp and HIC-purified 20kDa PEGbv. The overall yield, purity, and biological activity of the conjugate is compared to previously described TNFbp conjugates.

### Conjugate Preparation

The c105 30kDa TNFbp mutein used in this example was can be prepared as described in published PCT Publication No. WO 92/16221. Alternatively, the c105 mutein is prepared as follows. *E. Coli* cells expressing the c105 mutein are harvested by centrifugation. The cell sludge is adjusted to approximately 40% wet weight solids by the addition of purified water. The mixture is then further diluted with an equal volume of breaking buffer (50 mM Tromethamine, 4 mM EDTA, pH 7.2) to give a suspension with approximately 20% wet weight solids. The cell sludge is passed five times through a high pressure homogenizer operating at 5515,8 KPa (8,000 psi) to produce the cell homogenate. The homogenate is cooled to less than or equal to 10°C prior to each pass through the homogenizer. The homogenate was centrifuged and the solids fraction containing the c105 is retained. The solids are diluted and centrifuged again to give washed inclusion bodies.

The washed inclusion bodies are then dissolved by addition of 8 M urea and 150 mM cysteine in 50 mM TRIS, pH 9.5 This mixture is allowed to stir for two hours at room temperature prior to refolding. Under these conditions, the c105 mutein is denatured and reduced.

The reduced denatured c105 mutein is refolded by dilution with 1.1 M urea, 50 mM Tris to give a final refold solution comprised of 200ug/mL c105 mutein, 1.5 M urea, 7.5 mM cysteine, 50 mM Tris, pH 9.7. The refold mixture is held at 6-10°C for two days. Refold efficiency is monitored by reverse phase HPLC and cation exchange HPLC.

The refold mixture is then brought to pH 5.0 by addition of acetic acid and HCl. The refold mixture is loaded onto a cation exchange column (S-Sepharose big bead resin) previously equilibrated in 25 mM sodium acetate, 65 mM NaCl, pH 5 at 4°C. After loading, the column is washed with the same equilibration buffer. The column is eluted with a gradient from 65 to 350 mM NaCl in 25 mM sodium acetate, pH 5. The c105 mutein is eluted at about 200 mM NaCl and is collected in one pool.

The collected pool containing the c105 mutein is diluted with 1.5 volumes of 5 M NaCl, 40 mM sodium phosphate, adjusted to pH 6, and loaded onto a hydrophobic interaction column (Toyo Butyl 650 M column), previously equilibrated in 3 M NaCl, 20 mM sodium phosphate, pH 6. At the end of the load, the column is washed with equilibration buffer. The c105 mutein is eluted using a linear eight column volume decreasing salt gradient running from 3 M to 1 M NaCl, in 20 mM sodium phosphate, at pH 6. The c105 mutein is collected in one pool. The pool is then concentrated to approximately 3 g/L c105 mutein and then diafiltered against 20 mM sodium phosphate, pH 6.0 until the final conductivity is less than 4 mmho (approximately six volumes).

The diafiltered pool is loaded onto a SP-Sepharose high performance column equilibrated in 20 mM sodium phosphate, pH 6.0. After loading, the column is washed with additional equilibration buffer and eluted with a combination pH/salt gradient from 20 mM sodium phosphate, 50 mM NaCl, pH 6.0 to 20 mM sodium phosphate, 50 mM NaCl, pH 6.5. The c105 mutein elutes in the later half of the gradient at about 35 mM NaCl. The c105 mutein can be stored frozen at this point.

The 20kDa PEGbv used in the example was synthesized using the "One-step" procedure as described in Example 1 above. A portion of the synthesized 20kDa PEGbv was then HIC-purified as described in Example 4, Run 5. Both non-HIC-purified and HIC-purified 20kDa PEGbv was then used to prepare PEGbv:TNFbp conjugates. The conjugates were prepared as follows: the c105 30kDa TNFbp mutein was exposed to a 1.3-fold molar excess of DTT in 20 mM phosphate, pH 7.6, for 5-6 hours at 15°C in order to remove an extra cysteine attached to c105 during the refolding process. The pH was adjusted to 6.0 and the sample loaded onto a S-Sepharose Fast Flow column (at 4 - 6 mg/mL bed volume), washed with 3 column volumes of 20 mM phosphate buffer, pH 6.0 (to remove the DTT) and eluted with 20 mM phosphate, 70 mM NaCl, pH 6.0.

The reduced c105 30KDa TNFbp mutein solution (∼4.7 mg/mL) was then adjusted to pH 7.4 - 7.5 with 1M NaOH. HIC-purified or non-HIC-purified 20kDa PEGbv (0.9 mg per mg TNFbp) was added to the reduced c105 TNFbp with mixing. After mixing, the solution was allowed to stand at ambient temperature for 12 - 18 hours.

### Conjugate Analysis

### PEGylation Assay

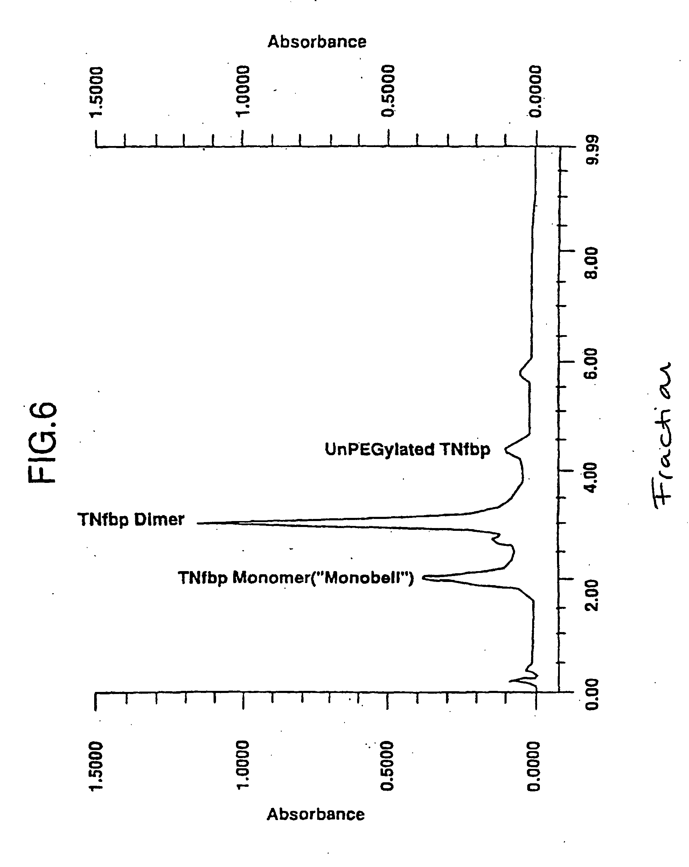

The conversion of TNFbp into 20kDa PEGbv:TNFbp dumbbell was quantitated using a POROS™ HS/H 4.6/50 cation exchange HPLC column (Perseptive Biosystems, Cambridge, MA). A 50 µl sample was loaded onto the column and a 10 minute, 20mM sodium acetate, pH 3.75, 0 - 1M NaCl gradient used to elute the sample from the column. A representative elution profile for a c105 30KDa TNFbp mutein monobell, i.e., PEGbv with c105 30KDa TNFbp attached at only one end, c105 30KDa TMFbp mutein dumbbell, and an unpegylated c105 30KDa TNFbp mutein sample was depicted in Figure 6. c105 30KDa TNFbp mutein monobell eluted first, followed by c105 30KDa TNFbp mutein dumbbell and unPEGylated c105 30KDa TNFbp mutein.

### Results

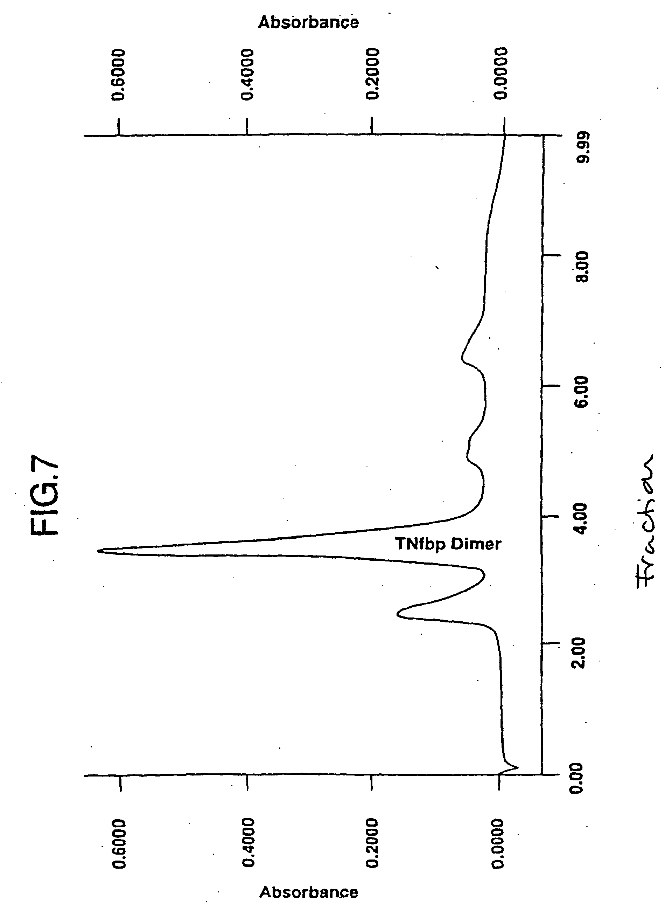

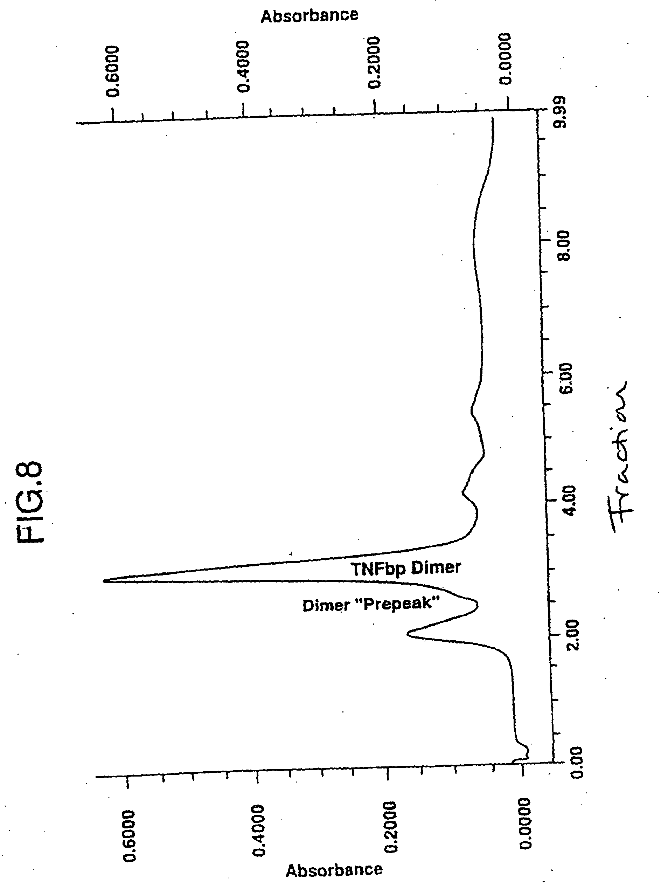

The percent conversion to TNFbp dumbbell for the HIC-purified material was 66.7%, with no detectable "prepeak" (see Figure 7). Non-HIC-purified PEGbv from the same lot gave only 52.9% conversion and had 6.7% "prepeak" (see Figure 8). This distinct "prepeak" is TNFbp dumbbell that is generated with high molecular weight PEGbv impurities.

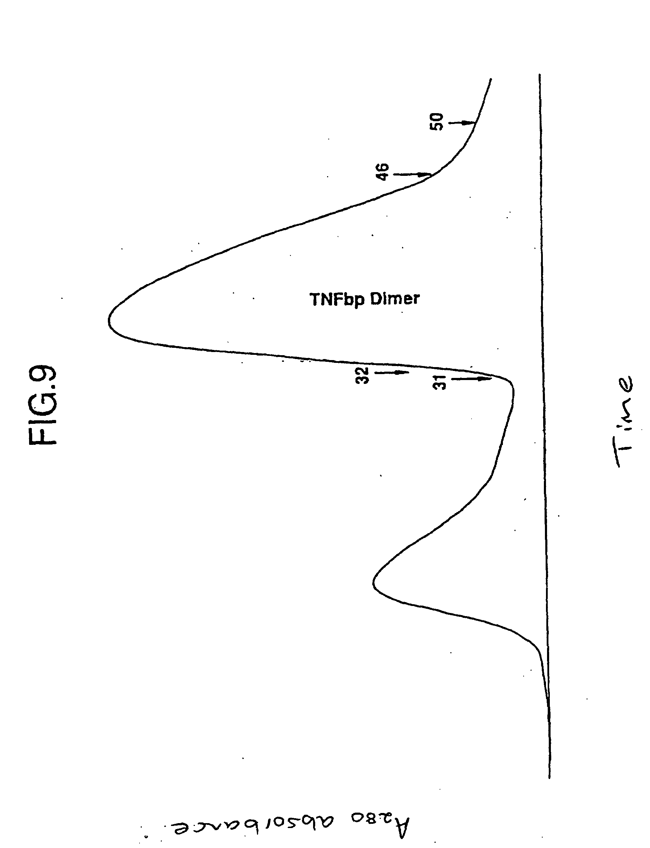

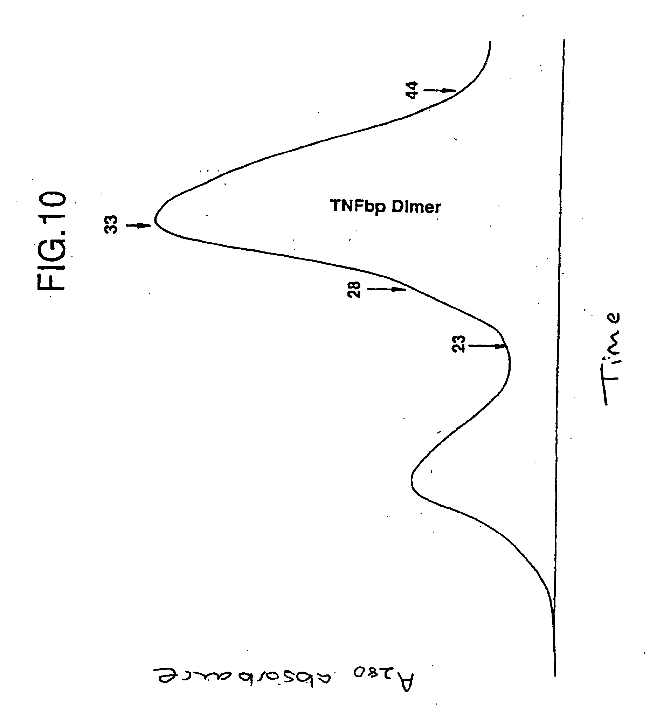

The PEGylation mixtures were then loaded onto 1.5 X 10 cm SP-Sepharose HP columns (4 mg/mL bed volume) equilibrated in 50mM sodium phosphate, pH 3.1. After loading, the column was washed with equilibration buffer and then the TNFbp dumbbell eluted using 20 column volumes of a 50mM sodium phosphate, 0.25M NaCl, pH 3.1 - 50mM sodium phosphate, 0.5M NaCl, pH 3.1 gradient. The elution profiles are shown in Figures 9 and 10. Pooling was done several different ways for each run. Table 3 shows the TNFbp dumbbell % yield and % purity for the various pooling criteria used.

**Table 3**

| TNFbp Dumbbell Sample | Pooled Fractions | % yield 20kDa PEGby | % purity 20kDa PEGby |
|---|---|---|---|
| HIC-purified PEGbv | 31 - 36 | 53.70% | 96.90% |
| | 32 - 46 | 53.30% | 97.40% |
| | 31 - 50 | 58.00% | 97.10% |
| | | | |
| Unpurified PEGbv | 23 - 44 | 55.30% | 83.50% |
| | 28 - 44 | 50.70% | 90.60% |
| | 33 - 44 | 35.50% | 97.30% |

The Table 3 data shows a clear benefit of PEGbv HIC purification on subsequent TNFbp dumbbell purity and yields. For example, in order to obtain 97% purity with the non-HIC-purified PEGbv, only 37% of the TNFbp dumbbell could be recovered on SP-Sepharose HP; whereas as much as 58% could be recovered with the HIC-purified material, i.e., HIC purification of 20kDa PEGbv can increase the recovery of TNFbp dumbbell by as much as 1.5 - 1.6 fold.

### EXAMPLE 6

In this example, the ability of the HIC resin to separate PEGs based on end-group functionality was evaluated.

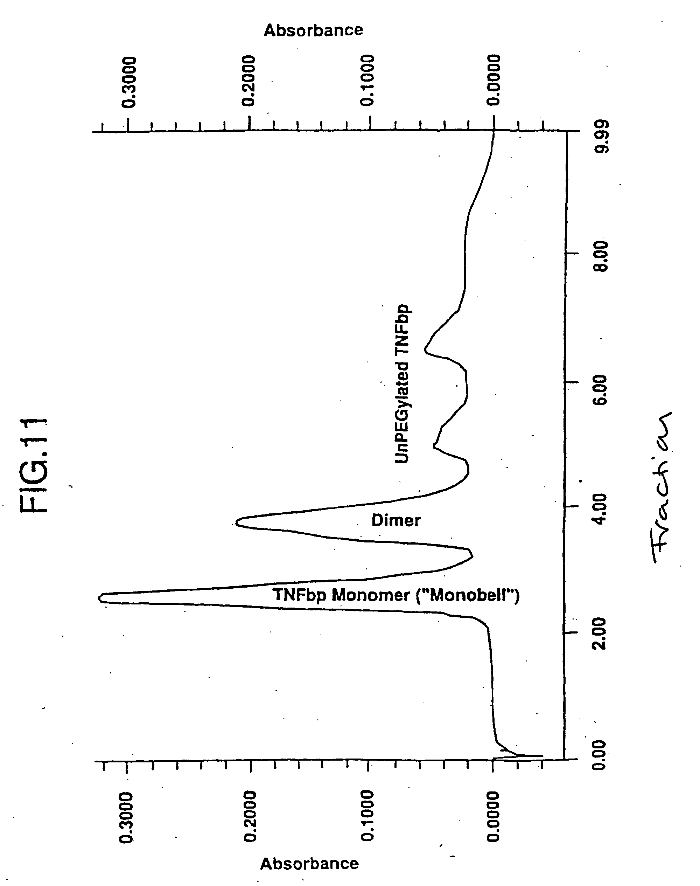

The "early eluting" material from Example 4, Run 3 was tested in the PEGylation assay. The "early eluting" material was those fractions which eluted just in front of the fractions which were pooled to provide the 20kDa PEGbv. And, as shown in Figure 11, this material, when used to make conjugates, made primarily TNFbp monobell. This indicates that the "early eluting" material contains predominantly PEG 20kDa mono-vinylsulfone, and thus demonstrates the ability of the Butyl HIC resin to separate 20kDa PEGbv based on end-group functionality.

### EXAMPLE 7

In this example, the stability of the linkage between the c105 TNFbp mutein and PEG-*bis*-vinyl sulfone was studied. Known amounts of the c105 TNFbp dumbbell were incubated in PBS, pH 7.4, at 37°C for up to one week with aliquots removed at intervals for analysis by SDS PAGE. Essentially no decomposition of the c105 TNFbp dumbbell was observed. At pH 10 at 37°C for 1 week, only 5-10% degradation of the conjugate was observed. In further stability studies, the activated PEG linkers prepared by the one-step method of Example 1 were compared to the activated PEG linkers prepared by the multi-step method as described in PCT/US95/07555.

First, the one-step and multi-step activated linkers were derivatized with 2-mercaptobenzoic acid to determine the stability of the linkers using UV. A 3.0 ml of a 1 mM 2-mercaptobenzoic acid (orthothiolbenzoic acid) was added to 0.5 ml of each linker solution (20 mg of linker were dissolved in 0.5 ml of 50 mM sodium phosphate, pH 7.5) and mixed briefly by vortexing. The solution was then incubated at room temperature for a minimum of 18 hours. Longer incubation times at room temperature (up to 48 hours) are acceptable.

The derivatized linker solutions were then diluted 1:10 in PBS and heat treated at 90°C for 1 hour. The heat treated linker solutions were removed from the heat and 100 µl to 200 µl of the solutions were injected onto a BIO SIL™ 250-5 column (BioRad, Hercules, California) and ran in 10 mM sodium phosphate, 150 mM. NaCl, 0.004% SDS, ph 6.5, at a flow rate of .5 ml/min. The collected samples were measured by UV at an O.D. of 254 nm. Tables 4 and 5 provide the results of the UV measurements. In these tables, the % change in product-UV peak is calculated based on the UV purity of the standard, which was not heat treated, minus the 90°C heat treated sample purity. Purity was calculated as the mean peak area divided by total peak area. The variability of the assay was 2%.

**Table 4**

| *"One-Step Synthesis"* | |
|---|---|
| Sample I.D. | Percent Change in Product UV Peak |
| 1 | -1.91 |
| 2 | 1,70 |
| 3 | 0.82 |
| 5 | 1.59 |
| 6 | 0.71 |
| 7 | 0.04 |
| 8 | -0.51 |
| 9 | 0.30 |
| 10 | 1.09 |
| 11 | 1.61 |
| 12 | 3.72 |
| Average | 0.83 |
| Standard Deviation | 1.43 |

**Table 5**

| *"Multi-Step Synthesis"* | |
|---|---|
| Sample I.D. | Percent Change in Product UV Peak |
| 002 | 9.28 |
| 006 | 10.75 |
| 007 | 13.33 |
| 008 | 13.95 |
| 009 | 20.58 |
| 010 | 14.41 |
| 014 | 11.37 |
| 015 | 9.85 |
| Average | 12.94 |
| Standard Deviation | 3.62 |

As shown in comparing the results of Tables 4 and 5, the one-step linker is more stable than the multi-step linker. The results also show that the change in stability of the one-step linker is within the 2% assay variability, whereas the change in stability of the four-step linker is greater than the 2% assay variability.

The foregoing description of the invention is exemplary for purposes of illustration and explanation.

## Claims

1. A method for making an activated polyethylene glycol linker comprising the steps of:
(a) obtaining a polyethylene glycol polymer (PEG) having a reactive hydroxyl group;
(b) converting said hydroxyl group to a reactive Michael acceptor to produce at least one form of an activated linker;
(c) applying said activated linker to a hydrophobic interaction chromatography (HIC) column that utilizes polymeric resin having a hydrophobic pendant group located on the surface of a hydrophilic vinyl copolymer backbone, wherein said hydrophobic pendant group is selected from octyl, butyl, phenyl or ether; and
(d) selectively isolating an activated linker form by utilizing elution conditions which allow for separation of various forms based on size and/or end-group functionality.

2. The method of claim 1, wherein the step (b) comprises:
(i) adding THF as the solvent to the compound having a reactive hydroxy group;
(ii) adding a reagent having a reactive carbonyl, nitrile or sulfone group; and
(iii) adding a base to form the reactive Michael acceptor.

3. The method of claim 1, wherein the elution conditions comprise utilizing a linear 5 - 0M sodium chloride gradient.

4. The method of claims 1-3, wherein said PEG has a molecular weight in the range of 2kDa to 100kDa.

5. The method of claim 4, wherein said PEG has a molecular weight of 3.4kDa, 5kDa, 10kDa or 20kDa.

6. The method of claim 5, wherein said PEG has a molecular weight of 20kDa.

7. The method of claims 1-6, wherein said PEG is PEG diol.

8. The method of any one of claims 2 to 7, wherein THF is added at a ratio in the range of 1:10 to 1:50 weight/volume of PEG to THF.

9. The method of claim 8, wherein said ratio is 1:30.

10. The method of claim 2, wherein the reagent having a reactive sulfone group is vinylsulfone.

11. The method of claim 2, wherein said base is potassium *tert*-butoxide.

12. The method of claim 2, wherein the reagent having a reactive sulfone group is divinylsulfone.

13. The method of claim 1, further comprising after step (a) : dissolving the PEG having a reactive hydroxy group in toluene; and drying said toluene solution prior to step (b).

14. The method of claims 1-13; wherein said HIC comprises a butyl or phenyl ligand on a methacrylic backbone.

15. A process for the preparation of a pegylated biologically active molecule, said process comprising:
(a) steps (a) to (d) of claim 1;
(b) reacting the HIC-purified PEG of step (a) in this claim with a biologically active molecule to produce a pegylated biologically active molecule.

16. A process according to Claim 15, wherein said reaction step comprises: mixing said HIC-purified PEG with said biologically active molecule; and allowing said mixture to stand at room temperature for 12 -18 hours.

17. A process according to Claim 15, wherein said molecule is a tumor necrosis factor (TNF) inhibitor.

18. A process according to Claim 17, wherein said TNF inhibitor is selected from the group consisting of a 30kDa TNF inhibitor, a 40kDa TNF inhibitor, or truncated variants or muteins thereof.

19. A process according to Claim 18, wherein said TNF inhibitor is a TNF inhibitor mutein.

20. A process according to Claim 19, wherein said TNF inhibitor mutein is c105 30kDa TNF inhibitor.

21. A process according to Claim 15, wherein said pegylated biologically active molecule has the formula R₁-X-R₂, wherein:
X comprises an HIC-purified PEG having a first reactive group and a second reactive group, and wherein said first reactive group is a Michael acceptor;
R₁ comprises a biologically active molecule having a reactive thiol moiety, said thiol moiety being covalently bonded with said Michael acceptor; and
R₂ comprises a molecule or a nonbiologically-active group bonded to said PEG by reaction with said second reactive group.

22. A process according to Claim 21, wherein X is polyethylene glycol-bis-vinyl sulfone and wherein R₁ and R₂ is c105 30kDa TNF inhibitor.

## Patentansprüche

1. Verfahren zur Herstellung eines aktivierten Polyethylenglykol-Linkers, welches die Schritte umfaßt:
(a) Erhalten eines Polyethylenglykol-Polymers (PEG) mit einer reaktiven Hydroxylgruppe;
(b) Umwandeln besagter Hydroxylgruppe in einen reaktiven Michael-Akzeptor, um wenigstens eine Form eines aktivierten Linkers zu erzeugen;
(c) Aufbringen besagten aktivierten Linkers auf eine Hydrophobchromatographie(HIC)-Säule, die polymeres Harz mit einer hydrophoben Seitengruppe, angeordnet auf der Oberfläche einer hydrophilen Vinyl-Copolymer-Hauptkette, verwendet, wobei besagte hydrophobe Seitengruppe ausgewählt ist aus Octyl, Butyl, Phenyl oder Ether; und
(d) selektives Isolieren einer aktivierten Linkerform durch Verwendung von Elutionsbedingungen, die Trennung verschiedener Formen auf der Grundlage von Größe und/oder Endgruppenfunktionalität ermöglichen.

2. Verfahren nach Anspruch 1, **dadurch gekennzeichnet, daß** Schritt (b) umfaßt:
(i) Zugeben von THF als dem Lösungsmittel zur Verbindung mit einer reaktiven Hydroxygruppe;
(ii) Zugeben eines Reagens mit einer reaktiven Carbonyl-, Nitril- oder Sulfongruppe; und
(iii) Zugeben einer Base, um den reaktiven Michael-Akzeptor zu bilden.

3. Verfahren nach Anspruch 1, **dadurch gekennzeichnet, daß** die Elutionsbedingungen die Verwendung eines linearen 5 - 0 M Natriumchlorid-Gradienten umfassen.

4. Verfahren nach den Ansprüchen 1-3, **dadurch gekennzeichnet, daß** besagtes PEG ein Molekulargewicht im Bereich von 2 kDa bis 100 kDa hat.

5. Verfahren nach Anspruch 4, **dadurch gekennzeichnet, daß** besagtes PEG ein Molekulargewicht von 3,4 kDa, 5 kDa, 10 kDa oder 20 kDa hat.

6. Verfahren nach Anspruch 5, **dadurch gekennzeichnet, daß** besagtes PEG ein Molekulargewicht von 20 kDa hat.

7. Verfahren nach den Ansprüchen 1-6, **dadurch gekennzeichnet, daß** besagtes PEG PEG-Diol ist.

8. Verfahren nach einem der Ansprüche 2 bis 7, **dadurch gekennzeichnet, daß** THF in einem Verhältnis von 1:10 bis 1:50 Gewicht/Volumen von PEG zu THF zugegeben wird.

9. Verfahren nach Anspruch 8, **dadurch gekennzeichnet, daß** besagtes Verhältnis 1:30 ist.

10. Verfahren nach Anspruch 2, **dadurch gekennzeichnet, daß** das Reagens mit einer reaktiven Sulfongruppe Vinylsulfon ist.

11. Verfahren nach Anspruch 2, **dadurch gekennzeichnet, daß** besagte Base Kalium-tertbutoxid ist.

12. Verfahren nach Anspruch 2, **dadurch gekennzeichnet, daß** das Reagens mit einer reaktiven Sulfongruppe Divinylsulfon ist.

13. Verfahren nach Anspruch 1, **dadurch gekennzeichnet, daß** es nach Schritt (a) weiter umfaßt: Lösen des PEGs mit einer reaktiven Hydroxygruppe in Toluol; und Trocknen besagter Toluol-Lösung vor Schritt (b).

14. Verfahren nach den Ansprüchen 1-13, **dadurch gekennzeichnet, daß** besagte HIC einen Butyl- oder Phenyl-Liganden auf einer Methacryl-Hauptkette umfaßt.

15. Verfahren zur Herstellung eines pegylierten, biologisch aktiven Moleküls, wobei besagtes Verfahren umfaßt:
(a) die Schritte (a) bis (d) von Anspruch 1;
(b) Umsetzen des HIC-gereinigten PEGs von Schritt (a) in diesem Anspruch mit einem biologisch aktiven Molekül, um ein pegyliertes, biologisch aktives Molekül herzustellen.

16. Verfahren nach Anspruch 15, **dadurch gekennzeichnet, daß** besagter Reaktionsschritt umfaßt: Mischen besagten HIC-gereinigten PEGs mit besagtem biologisch aktiven Molekül; und Stehenlassen besagter Mischung bei Raumtemperatur für 12 - 18 Stunden.

17. Verfahren nach Anspruch 15, **dadurch gekennzeichnet, daß** besagtes Molekül ein Tumornekrosefaktor(TNF)-Inhibitor ist.

18. Verfahren nach Anspruch 17, **dadurch gekennzeichnet, daß** besagter TNF-Inhibitor ausgewählt ist aus der Gruppe, die aus einem 30kDa-TNF-Inhibitor, einem 40-kDa-TNF-Inhibitor oder verkürzten Varianten oder Muteinen derselben besteht.

19. Verfahren nach Anspruch 18, **dadurch gekennzeichnet, daß** besagter TNF-Inhibitor ein TNF-Inhibitor-Mutein ist.

20. Verfahren nach Anspruch 19, **dadurch gekennzeichnet, daß** besagtes TNF-Inhibitor-Mutein c105-30kDa-TNF-Inhibitor ist.

21. Verfahren nach Anspruch 15, **dadurch gekennzeichnet, daß** besagtes pegyliertes, biologisch aktives Molekül die Formel R₁-X-R₂ hat, wobei: X ein HIC-gereinigtes PEG mit einer ersten reaktiven Gruppe und einer zweiten reaktiven Gruppe umfaßt, und wobei besagte erste reaktive Gruppe ein Michael-Akzeptor ist;
R₁ ein biologisch aktives Molekül mit einer reaktiven Thiol-Einheit ist, wobei besagte Thiol-Einheit kovalent an besagten Michael-Akzeptor gebunden ist; und
R₂ ein Molekül oder eine biologisch nicht-aktive Gruppe umfaßt, die an besagtes PEG durch Reaktion mit besagter zweiten reaktiven Gruppe gebunden ist.

22. Verfahren nach Anspruch 21, **dadurch gekennzeichnet, daß** X Polyethylenglykol-bisvinylsulfon ist und R₁ und R₂ c105-30kDa-TNF-Inhibitor ist.

## Revendications

1. Procédé pour réaliser un lieur activé de polyéthylèneglycol, comprenant les étapes de :
(a) obtenir un polymère de polyéthylèneglycol (PEG) ayant un groupe hydroxyle réactif ;
(b) convertir ledit groupe hydroxyle en un accepteur réactif de Michael pour produire au moins une forme d'un lieur activé ;
(c) appliquer ledit lieur activé à une colonne de chromatographie par interaction hydrophobe (HIC) qui utilise de la résine polymère ayant un groupe latéral hydrophobe situé sur la surface d'une ossature de copolymère de vinyle hydrophile, où ledit groupe latéral hydrophobe est choisi à partir d'octyle, de butyle, de phényle ou d'éther ; et
(d) isoler de façon sélective une forme de lieur activé en utilisant des conditions d'élution qui permettent la séparation de différentes formes en se basant sur la taille et/ou la fonctionnalité du groupe terminal.

2. Procédé selon la revendication 1, dans lequel l'étape (b) comprend :
(i) ajouter THF comme solvant au composé ayant un groupe hydroxy réactif ;
(ii) ajouter un réactif ayant un groupe réactif carbonyle, nitrile ou sulfone ; et
(iii) ajouter une base pour former un accepteur réactif de Michael.

3. Procédé selon la revendication 1, dans lequel les conditions d'élution comprennent l'utilisation d'un gradient linéaire de chlorure de sodium 5 - 0 M.

4. Procédé selon les revendications 1 - 3, dans lequel ledit PEG présente un poids moléculaire dans la plage de 2 kDa à 100 kDa.

5. Procédé selon la revendication 4, dans lequel ledit PEG présente un poids moléculaire de 3,4 kDa, 5kDa, 10 kDa ou 20 kDa.

6. Procédé selon la revendication 5, dans lequel ledit PEG présente un poids moléculaire de 20 kDa.

7. Procédé selon les revendications 1 - 6, dans lequel ledit PEG est PEG diol.

8. Procédé selon une quelconque des revendications 2 à 7, dans lequel THF est ajouté dans un rapport dans la plage de 1:10 à 1:50 poids/volume de PEG à THF.

9. Procédé selon la revendication 8, dans lequel ledit rapport est 1:30.

10. Procédé selon la revendication 2, dans lequel le réactif ayant un groupe réactif de sulfone est de la vinylsulfone.

11. Procédé selon la revendication 2, dans lequel ladite base est du tert-butoxyde de potassium.

12. Procédé selon la revendication 2, dans lequel le réactif ayant un groupe réactif de sulfone est de la divinylsulfone.

13. Procédé selon la revendication 1, comprenant de plus après l'étape (a) : dissoudre le PEG ayant un groupe hydroxy réactif dans du toluène ; et sécher ladite solution de toluène avant l'étape (b).

14. Procédé selon les revendications 1 - 13, dans lequel ladite HIC comprend un ligand de butyle ou de phényle sur une ossature méthacrylique.

15. Procédé pour la préparation d'une molécule pégylée biologiquement active, ledit procédé comprenant :
(a) les étapes (a) à (d) de la revendication 1,
(b) faire réagir PEG purifié par HIC de l'étape (a) de cette revendication avec une molécule biologiquement active pour produire une molécule pégylée biologiquement active.

16. Procédé selon la revendication 15, dans lequel ladite étape réactionnelle comprend : mélanger ledit PEG purifié par HIC avec ladite molécule biologiquement active ; et laisser ledit mélange à température ambiante pendant 12 - 18 heures.

17. Procédé selon la revendication 15, dans lequel ladite molécule est un inhibiteur du facteur de nécrose tumorale (TNF).

18. Procédé selon la revendication 17, dans lequel ledit inhibiteur de TNF est choisi dans le groupe constitué d'un inhibiteur de TNF de 30 kDa, un inhibiteur de TNF de 40 kDa ou de mutéines ou variants tronqués de ceux-ci.

19. Procédé selon la revendication 18, dans lequel ledit inhibiteur de TNF est une mutéine inhibitrice de TNF.

20. Procédé selon la revendication 19, dans lequel ladite mutéine inhibitrice de TNF est l'inhibiteur de TNF de 30 kDa c105.

21. Procédé selon la revendication 15, dans lequel ladite molécule pégylée biologiquement active présente la formule R₁-X-R₂, où :
X comprend un PEG purifié par HIC ayant un premier groupe réactif et un second groupe réactif ; et où ledit premier groupe réactif est un accepteur de Michael ;
R₁ comprend une molécule biologiquement active ayant une fraction de thiol réactive, ladite fraction de thiol étant liée de façon covalente audit accepteur de Michael ; et
R₂ comprend une molécule ou un groupe non biologiquement actif lié audit PEG par réaction avec ledit second groupe réactif.

22. Procédé selon la revendication 21, dans lequel X est de la polyéthylèneglycol-bis-vinylsulfone, et dans lequel R₁ et R₂ sont l'inhibiteur de TNF de 30 kDa c105.
